# EUROPEAN PATENT APPLICATION

(11) **EP 1 106 167 A2**
(43) Date of publication of application: **13.06.2001**
(21) Application number: 00310764.6
(22) Date of filing: 04.12.2000
(51) Int. Cl.: A61K 7/13

(54) **Dye compositions for keratin fibers comprising a nonionic compound**

(30) Priority: 03.12.1999 JP 34554699
(71) Applicant: L'OREAL, 75008 Paris (FR)
(72) Inventor: Bone, Eric, 92500 Rueil Malmaison (FR); Mori, Harumi, Chofu-shi, Tokyo 182-0014 (JP); Yamada, Hidetoshi, Fuchu-shi, Tokyo 183-0053 (JP)
(74) Representative: Ellis-Jones, Patrick George Armine

(57) **Abstract**

The present invention relates to a dye composition for keratin fibers, in particular for human keratin fibers such as hair, comprising, at lest one dye [oxidation dye (base and/or coupler) or direct dye], and at least one nonionic compound of the general formula R-(OCH₂CH₂)ₙ-OR¹, wherein R denotes a C₁₀-C₃₀ alkyl radical, R' denotes a C₁₀-C₃₀ alkyl radical which may be substituted with a hydroxyl radical, and n is an integer from 1 to approximately 100.

The present invention also relates to processes and devices for dyeing using the aforesaid compositions.

## Description

The present invention relates to dye compositions for keratin fibers, in particular for human keratin fibers such as hair, comprising at lest one colorant [oxidation dye (base and/or coupler) or direct dye], and at least one nonionic compound of the general formula R-(OCH₂CH₂)ₙ-OR', wherein R denotes a C₁₀-C₃₀ alkyl radical, R' denotes a C₁₀-C₃₀ alkyl radical which may be substituted by a hydroxyl radical, and n is an integer from 1 to approximately 100.

Two types of coloration can be distinguished in the field of hair treatment.

The first is semi-permanent or temporary coloration, or direct coloration, which involves dyes capable of giving the hair's natural color a more or less pronounced color change which may withstand shampooing several times. These colorants are known as direct dyes; they can be used with or without an oxidizing agent. In the presence of an oxidizing agent, the aim is to obtain a lightening direct coloration. The lightening coloration is carried out by applying to the hair the mixture, prepared at the time of use, of a direct dye and an oxidizing agent, and in particular makes it possible to obtain, by lightening the melanin in the hair, an advantageous effect such as a unified color in the case of gray hair, or to bring out the color in the case of naturally pigmented hair.

The second is permanent coloration or oxidation coloration. This is carried out with dyes known as "oxidation" dyes comprising oxidation dye precursors and couplers. Oxidation dye precursor, commonly known as "oxidation bases", are initially colorless or weakly colored compounds which develop their dyeing power on the hair in the presence of oxidizing agents added at the time of use, leading to the formation of colored compounds and dyes. The formation of these colored compounds and dyes results either from an oxidative condensation of the "oxidation bases" with themselves or from an oxidative condensation of the "oxidation bases" with coloration modifier compounds commonly known as "couplers", which are generally present in the dye compositions used in oxidation coloration.
The variety of the molecules employed therein, constituted on the one hand by the oxidation bases and on the other hand by the couplers, makes it possible to obtain a pallet very rich in color.
In order to further vary the shades obtainable by the oxidation dyes, or to enrich the glints, direct dyes may be added thereto.

In order to localize the dye product as applied on the hair in order that the dye product does not touch the face or the area outside the area to be dyed, traditional thickeners such as crosslinked polyacrylic acid, hydroxyethylcelluloses, waxes, mixtures of nonionic surfactants of HLB (Hydrophilic Lipophilic Balance), which, chosen as appropriate, provide the gelling effect when diluted by water and/or surfactants, have been conventionally used.

Thus, the applicant has discovered that the ingredients of the traditional thickener type, surfactant and solvent generally decreased the holding of the dye on the fibers, which results in dull shades and also larger quantity of the dye, solvent and/or surfactant to be used to dissolve the dye, if stronger shades are needed.
Furthermore, it was also discovered that after mixing with oxidizing agent, the dyeing compositions containing one or more dyes and the above-described ingredients lost their gelling character and exhibited undesirable fluidity.

After intensive research conducted on this problem, the Applicant has discovered that it is possible to obtain the coloring compositions which do not flow and remain more localized at the location where it is applied, and which permits to obtain stronger and more chromatic (luminous) shades, if an effective amount of the particular nonionic new compound is introduced in the dyeing composition, or in the oxidizing composition (when it relates to oxidation dyeing or lightening direct dyeing), or in the two compositions at the same time.

This discovery forms the basis of the present invention.

The present invention thus has an object to provide a dyeing composition for keratin fibers, particularly for human keratin fibers such as hair, comprising, in a medium suitable for dyeing, at least one dye, and characterized by the fact that it further contains at least one nonionic compound of the general formula R-(OCH₂CH₂)ₙ-OR', wherein R denotes a C₁₀-C₃₀ alkyl radical, R' denotes a C₁₀-C₃₀ alkyl radical which may be substituted by a hydroxyl radical , and n is an integer from 1 to approximately 100.

According to the present invention, it is possible to provide a dyeing composition having high viscosity by utilizing a small amount of nonionic compound according to the invention, and to improve the quality of use at application.
It is also possible in an advantageous manner to reduce the consumption of the surfactants.
The invention also makes it possible to reduce the quantity of the active colorants used in the dyeing composition compared with the classic technique in the prior art.

Furthermore, the composition according to the invention makes it possible to obtain shades with little selectivity and good resistance with respect to chemical agents (shampoos, permanent agents) or natural agents (light, perspiration).

Another object of the present invention is to provide a composition ready to use for dyeing keratin fibers, constituted with a composition A comprising, in a medium appropriate for dyeing, at least one oxidation dye (base and/or coupler), or at least one direct dye, and a composition B comprising at least one oxidizing agent, the nonionic compound as defined above being present in the composition A or in the composition B, or in both of the compositions A and B.

The present invention also provides a dyeing process of keratin fibers, particularly human keratin fibers such as hair, consisting of applying to the dry or wet keratin fibers at least one composition containing, in a medium appropriate for dyeing, at least one direct dye and at least one nonionic compound as defined above, and after having optionally left the composition to act on the fibers for a resting time from 3 to 60 minutes, rinsing the fibers, optionally washing the fibers, rinsing again and then drying the fibers.

The present invention also relates to the dyeing process of keratin fibers, particularly human keratin fibers such as hair, consisting of applying on the fibers at least one composition A containing, in a medium appropriate for dyeing, at least one oxidation dye (base and/or coupler) or at least one direct dye, the color being developed at alkaline, neutral or acid pH, with the aid of an oxidizing composition B which is mixed just at the moment of use with the composition A or which is applied sequentially without intermediate rinsing, the nonionic compound defined according to the present invention being present in either the composition A or the composition B, or in both of the compositions A and B.

The present invention relates particularly to a dyeing process which consists of applying the composition for ready to use, extemporarily prepared at the moment of use from the compositions (A) and (B) described above, on the dry or wet keratin fibers, leaving it to act for a resting time varying preferably from 1 to 60 minutes approximately, and more preferably from 10 to 45 minutes approximately, rinsing the fibers, then optionally shampooing the fibers, and again rinsing and drying the fibers.

A variation of the process consists of taking a composition A' containing at least one oxidation dye and/or at least one direct dye, but without the nonionic compound of the invention, and another composition A" containing at least one nonionic compound of the invention, and mixing the compositions A' and A" and the oxidizing composition B at the moment of use, and applying the mixture thus obtained.

The invention also has an object to provide a dyeing device or "kit" having a plurality of compartments.

A device having 2 compartments according to the present invention comprises a compartment containing a composition A1 containing, in a medium appropriate for dyeing, at least one oxidation dye (base and/or coupler), or at least one direct dye, and another compartment containing a composition B1 containing, in a medium appropriate for dyeing, an oxidizing agent, the or those nonionic compounds defined above being present in either the composition Al or the composition B1, or in both of the compositions A1 and B1.

Another device having 3 compartments according to the invention comprises a first compartment containing a composition A2 containing, in a medium appropriate for dyeing, at least one oxidation dye (base and/or coupler), at least one direct dye, a second compartment containing a composition B2 containing, in a medium appropriate for dyeing, an oxidizing agent, and a third compartment containing a composition C containing, in a medium appropriate for dyeing, at least one nonionic compound defined above, wherein the composition A2 and/or the composition B2 can also contain the above-defined nonionic compound.

The present invention also relates to the use of the above-defined dyeing compositions or dyeing device or "kit" having a plural of compartments for dyeing keratin fibers such as human hair.

However, other characteristics, aspects, objects and advantages of the invention will become more clear by reading the description and the examples which follow hereinbelow.

The nonionic compound according to the present invention is preferably a compound of the formula R-(OCH₂CH₂)ₙ-OR' wherein R denotes a C₁₆-C₁₈ alkyl radical, R' denotes a C₁₆-C₁₈ alkyl radical which may be substituted by a hydroxyl radical, and n is an integer equal to or larger than 20.
The nonionic compound is more particularly again a compound of the formula R-(OCH₂CH₂)ₙ-OR' wherein R and R' denote a C₁₆-C₁₈ alkyl radical, and n is equal to
4. Such a compound is for example described in the CTFA dictionary under the name of PEG 4 DITTALLOW ETHER.
The nonionic compound is also more particularly again a compound of the formula R-(OCH₂CH₂)ₙ-OR' wherein R denotes a C₁₆-C₁₈ alkyl radical, R' denotes a substituted alkyl radical -C₁₄ OH, and n is preferably equal to 60.
Such a compound is for example found in the CTFA dictionary under the name of CETEARETH 60 MYRISTYL GLYCOL or HYDROGENATED TALLOWETH 60 MYRISTYL GLYCOL. CETEARETH 60 MYRISTYL GLYCOL is for example sold by the company AKZO under the commercial name ELFACOS GT 282 S.

The nonionic compound according to the invention is preferably used in a quantity which may vary between 0.05 and 10 wt % approximately of the total weight of the dyeing composition applied on the fibers. More preferably, the quantity varies between 0.1 and 5 wt % approximately.

The oxidation dyes which can be used according to the present invention are selected from the oxidation bases and/or the couplers.
Preferably, the compositions according to the invention contain at least one oxidation base.

The oxidation bases usable in the context of the present invention are chosen from those conventionally known as oxidation dye, from which we can cite paraphenylenediamines, double bases, ortho- and para-aminophenols and heterocyclic bases as follows;
- (I) paraphenylenediamines of the formula (I) below and their addition salts with an acid: 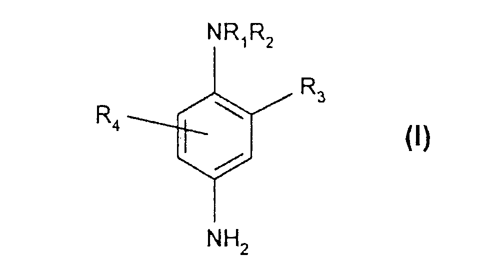 in which:
   R₁ represents a hydrogen atom, a C₁-C₄ alkyl radical, a monohydroxy (C₁-C₄ alkyl) radical, a polyhydroxy (C₂-C₄ alkyl) radical, a (C₁-C₄) alkoxy(C₁-C₄)alkyl radical, a C₁-C₄ alkyl substituted with a nitrogen-containing group, a phenyl radical or a 4'-aminophenyl radical;
   R₂ represents a hydrogen atom, a C₁-C₄ alkyl radical, a monohydroxy (C₁-C₄ alkyl) radical, a polyhydroxy (C₂-C₄ alkyl) radical, a (C₁-C₄) alkoxy(C₁-C₄)alkyl radical or a C₁-C₄ alkyl radical substituted with a nitrogen-containing group;
   R₁ and R₂ may also form with the nitrogen atom to which they are bonded a nitrogen-containing heterocycle ring of 5 or 6 members optionally substituted with one or more alkyl, hydroxy or ureido groups;
   R₃ represents a hydrogen atom, a halogen atom such as a chlorine atom, a C₁-C₄ alkyl, sulfo, carboxy, monohydroxy (C₁-C₄ alkyl) or hydroxy (C₁-C₄ alkoxy), acetylamino(C₁-C₄ alkoxy), mesylamino (C₁-C₄ alkoxy) or carbamoylamino (C₁-C₄ alkoxy) radical;
   R₄ represents a hydrogen atom, a halogen atom or a C₁-C₄ alkyl radical.

   Among the nitrogen-containing groups of formula (I) above, amino, mono (C₁-C₄)alkylamino, (C₁-C₄)dialkylamino, (C₁-C₄)trialkylamino, monohydroxy(C₁-C₄)alkylamino, imidazolinium and ammonium radicals may particularly be cited.
   Among the para-phenylenediamines of formula (I) above, para-phenylenediamine, para-toluylenediamine, 2-chloro-para-phenylenediamine, 2,3-dimethyl-para-phenylenediamine, 2,6-dimethyl-para-phenylenediamine, 2,6-diethyl-para-phenylenediamine, 2,5-dimethyl-para-phenylenediamine, N,N-dimethyl-para-phenylenediamine, N,N-diethyl-para-phenylenediamine, N,N-dipropyl-para-phenylenediamine, 4-amino-N,N-diethyl-3-methylaniline, N,N-bis(β-hydroxyethyl)-para-phenylenediamine, 4-amino-N,N-bis(β -hydroxyethyl)-3-methylaniline, 4-amino-3-chloro-N,N-bis(β-hydroxyethyl)-aniline, 2-β-hydroxyethyl-para-phenylenediamine, 2-fluoro-para-phenylenediamine, 2-isopropyl-para-phenylenediamine, N-(β-hydroxypropyl)-para-phenylenediamine, 2-hydroxymethyl-para-phenylenediamine, N,N-dimethyl-3-methyl-para-phenylenediamine, N,N-(ethyl-β -hydroxyethyl)-para-phenylenediamine, N-(β, γ -dihydroxypropyl)-para-phenylenediamine, N-(4'-aminophenyl)-para-phenylenediamine, N-phenyl-para-phenylenediamine, 2-β -hydroxyethyloxy-para-phenylenediamine, and their addition salts with an acid may particularly be cited.
   Among the para-phenylenediamines of formula (I) above, particularly preferred are para-phenylenediamine, para-toluylenediamine, 2-isopropyl-para-phenylenediamine, 2-β-hydroxyethyl-para-phenylenediamine, 2-β -hydroxyethyloxy-para-phenylenediamine, 2,6-dimethyl-para-phenylenediamine, 2,6-diethyl-para-phenylenediamine, 2,3-dimethyl-para-phenylenediamine, N,N-bis(β -hydroxyethyl)-para-phenylenediamine, 2-chloro-para-phenylenediamine, and their addition salts with an acid.
- (II) According to the invention, the double bases are understood to mean the compounds containing at least two aromatic rings on which amino and/or hydroxyl groups are carried.
   Among the double bases which can be used as oxidation bases in the dyeing compositions in accordance with the invention, there may be mentioned in particular the compounds corresponding to the following formula (II), and their addition salts with an acid: 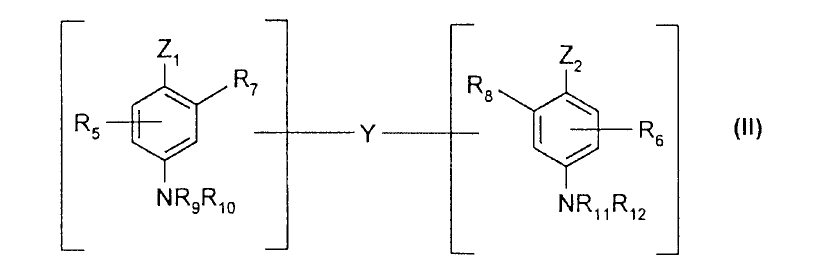 in which:
   - Z₁ and Z₂, identical or different, represent a hydroxyl or -NH₂ radical which may be substituted with a C₁-C₄ alkyl radical or by a linking arm Y;
   - the linking arm Y represents a linear or branched alkylene chain comprising from 1 to 14 carbon atoms, which may be interrupted by or which may end with one or more nitrogen-containing groups and/or one or more heteroatoms such as oxygen, sulphur or nitrogen atoms, and optionally substituted with one or more hydroxyl or C₁-C₆ alkoxy radicals;
   - R₅ and R₆ represent a hydrogen or halogen atom, a C₁-C₄ alkyl radical, a monohydroxy(C₁-C₄ alkyl) radical, a polyhydroxy(C₂-C₄ alkyl) radical, an amino(C₁-C₄ alkyl) radical or a linking arm Y;
   - R₇, R₈, R₉, R₁₀, R₁₁ and R₁₂, which are identical or different, represent a hydrogen atom, a linking arm Y or a C₁-C₄ alkyl radical;
   it being understood that the compounds of formula (II) contain only one linking arm Y per molecule.
   Among the nitrogen-containing groups of formula (II) above, there may be mentioned in particular the amino, mono (C₁-C₄)alkylamino, (C₁-C₄)dialkylamino, (C₁-C₄)trialkylamino, monohydroxy(C₁-C₄)alkylamino, imidazolinium and ammonium radicals.
   Among the double bases of formulae (II) above, reference is made more particularly to N, N'-bis(β-hydroxyethyl)-N,N'-bis(4'-aminophenyl)-1,3-diaminopropanol, N,N'-bis(β-hydroxyethyl)-N,N'-bis(4'-aminophenyl)ethylenediamine, N,N'-bis(4-aminophenyl) tetramethylenediamine, N,N'-bis(β -hydroxyethyl)-N,N'-bis(4-aminophenyl)tetramethylenediamine, N,N'-bis(4-methylaminophenyl)tetramethylenediamine, N,N'-bis(ethyl)-N,N'-bis(4'-amino-3'-methylphenyl)ethylenediamine, 1,8-bis(2,5-diaminophenoxy)-3,5-dioxaoctane, and their addition salts with an acid.
   Among the double bases of formulae (II), N,N'-bis(β -hydroxyethyl)-N,N'-bis(4'-aminophenyl)-1,3-diaminopropanol, 1,8-bis(2,5-diaminophenoxy)-3,5-dioxaoctane or one of their addition salts with an acid are particularly preferred.
- (III) The para-aminophenols corresponding to the formula (III) below and their addition salts with an acid. 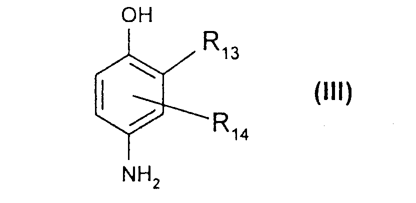 wherein:
   R₁₃ represents a hydrogen atom, a halogen atom such as fluorine, a C₁-C₄ alkyl radical, monohydroxy(C₁-C₄ alkyl), (C₁-C₄)alkoxy(C₁-C₄)alkyl or amino(C₁-C₄ alkyl), or hydroxy(C₁-C₄)alkylamino(C₁-C₄ alkyl) radical.
   R₁₄ represents a hydrogen atom or halogen atom such as fluorine, a C₁-C₄ alkyl radical, monohydroxy(C₁-C₄ alkyl), polyhydroxy (C₂-C₄ alkyl), amino (C₁-C₄ alkyl), cyano (C₁-C₄ alkyl) or (C₁-C₄)alkoxy(C₁-C₄ alkyl) radical,

   with the condition that at least one of radicals R₁₃ and R₁₄ represents a hydrogen atom.
   Among the para-aminophenols of formula (III) above, there may be mentioned more particularly para-aminophenol, 4-amino-3-methylphenol, 4-amino-3-fluorophenol, 4-amino-3-hydroxymethylphenol, 4-amino-2-methylphenol, 4-amino-2-hydroxymethylphenol, 4-amino-2-methoxymethylphenol, 4-amino-2-aminomethylphenol, 4-amino-2-(β-hydroxyethylaminomethyl)phenol, and their addition salts with an acid.
- (IV) The ortho-aminophenols which can be used as the oxidation bases in the context of the present invention, are in particular chosen from 2-aminophenol, 2-amino-1-hydroxy-5-methylbenzene, 2-amino-1-hydroxy-6-methylbenzene, 5-acetamido-2-aminophenol, and their addition salts with an acid.
- (V) Among the heterocyclic bases which can be used as the oxidation bases in the dyeing composition constituting the invention, pyridine derivatives, pyrimidine derivatives, pyrazole derivatives and their addition salts with an acid may be more particularly cited.
   Among the pyridine derivatives, there may be mentioned more particularly the compounds described for example in patents GB 1 026 978 and GB 1 153 196, such as 2,5-diaminopyridine, 2-(4-methoxypenyl)amino-3-aminopyridine, 2,3-diamino-6-methoxypyridine, 2-(β -methoxyethyl)amino-3-amino-6-methoxypyridine, 3,4-diaminopyridine and their addition salts with an acid.
   Among the pyrimidine derivatives, there may be mentioned more particularly the compounds described for example in German patent DE 2 359 399 or Japanese patents JP 88-169571 and JP 91-10659 or patent application WO 96/15765, such as 2,4,5,6-tetra-aminopyrimidine, 4-hydroxy-2,5,6-triaminopyrimidine, 2-hydroxy-4,5,6-triaminopyrimidine, 2,4-dihydroxy-5,6-diaminopyrimidine, 2,5,6-triaminopyrimidine, and such pyrazolo-pyrimidine derivatives as those described in the patent application FR-A-2 750 048 and among which mention is made of pyrazolo[1,5-a]pyrimidine-3,7-diamine; 2,5-dimethylpyarzolo[1,5-a]pyrimidine-3,7-diamine; pyrazolo[1,5-a]pyrimidine-3,5-diamine; 2,7-dimethylpyrazolo[1,5-a]pyrimidine-3,5-diamine; 3-aminopyrazolo[1,5-a]pyrimidin-7-ol; 3-aminopyrazolo[1,5-a]pyrimidin-5-ol; 2-(3-amino-pyrazolo[1,5-a]pyrimidin-7-ylamino)ethanol, 2(7-aminopyrazolo[1,5-a]pyrimidin-3-ylamino)ethanol, 2[(3-aminopyrazolo[1,5-a]pyrimidin-7-yl)-(2-hydroxy-ethyl)amino]ethanol, 2-[(7-aminopyrazolo-[1,5-a]pyrimidin-3-yl)-(2-hydroxyethyl)amino]ethanol, 5,6-dimethylpyrazolo[1,5-a]pyrimidine-3,7-diamine, 2,6-dimethylpyrazolo[1,5-a]pyrimidine-3,7-diamine, 2,5,N7,N7-tetramethylpyrazolo[1,5-a]pyrimidine-3,7-diamine, and the addition salts thereof and tautomeric forms thereof, when a tautomeric equilibrium exists, and the addition salts thereof with an acid.
   Among the pyrazole derivatives, one can more particularly cite the compounds described in patent DE 3 843 892, DE 4 133 957, and patent applications WO 94/08969, WO 94/08970, FR-A-2 733 749 and DE 195 43 988 such as 4,5-diamino-1-methylpyrazole, 4,5-diamino-1-hydroxyethylpyrazole, 3,4-diamiopyrazole, 4,5-diamino-1-(4'-chlorobenzyl)pyrazole, 4,5-diamino-1,3-dimethylpyrazole, 4,5-diamino-3-methyl-1-phenylpyrazole, 4,5-diamino-1-methyl-3-phenylpyrazole, 4-amino-1,3-dimethyl-5-hydrazinopyrazole, 1-benzyl-4,5-diamino-3-methylpyrazole, 4,5-diamino-3-tert-butyl-1-methylpyrazole, 4,5-diamino-1-tert-butyl-3-methylpyrazole, 4,5-diamino-1-(β -hydroxyethyl)-3-methylpyrazole, 4,5-diamino-1-ethyl-3-methylpyrazole, 4,5-diamino-1-ethyl-3-(4'-metoxyphenyl)pyrazole, 4,5-diamino-1-ethyl-3-hydroxymethylpyrazole, 4,5-diamino-3-hydroxymethyl-1-methylpyrazole, 4,5-diamino-3-hyroxymethyl-1-isopropylpyrazole, 4,5-diamino-3-methyl-1-isopropylpyrazole, 4-amino-5-(2'-aminoethyl)amino-1,3-dimethylpyrazole, 3,4,5-triaminopyrazole, 1-methyl-3,4,5-triaminopyrazole, 3,5-diamino-1-methyl-4-methylaminopyrazole, 3,5-diamino-4-(β-hydroxyethyl)amino-1-methylpyrazole, and their addition salts with an acid.

In accordance with the present invention, the oxidation bases represent preferably from 0.0005 to 12 wt% of the total weight of the composition (A).

The couplers which can be used in the dyeing process of the invention are those which have been conventionally used in the oxidation dyeing composition, that is, meta-phenylenediamines, meta-aminophenols and meta-diphenols, the mono- or poly-hydroxylated derivatives of naphthalene, sesamol and its derivatives and heterocyclic compounds such as for example indole couplers, indoline couplers, pyridine couplers and their addition salts with an acid.

Those couplers may be in particular chosen from 2-methyl-5-aminophenol, 5-N-(β -hydroxyethyl)amino-2-methylphenol, 3-aminophenol, 1,3-dihydroxybenzene, 1,3-dihydroxy-2-methylbenzene, 4-chloro-1,3-dihydroxybenzene, 2,4-diamino-1-(β -hydroxyethyloxy)benzene, 2-amino-4-(β-hydroxyethylamino)-1-methoxybenzene, 1,3-diaminobenzene, 1,3-bis(2,4-diaminophenoxy)propane, sesamol, α-naphthol, 6-hydroxyindole, 4-hydroxyindole, 4-hydroxy-N-methylindole, 6-hydroxyindoline, 2,6-dihydroxy-4-methylpyridine, 1-H-3-methylpyrazole-5-one, 1-phenyl-3-methylpyrazole-5-one, and their addition salts with an acid.

When these couplers are present, they preferably represent from 0.0001 to 10 % by weight approximately of the total weight of composition (A), and more preferably 0.005 to 5 % by weight approximately thereof.

In a generally known manner, the addition salts with an acid of the oxidation bases and couplers can be in particular chosen from hydrochlorides, hydrobromides, sulphates and tartrates, lactates and acetates.

The direct dye which can be used in the present invention are those which have conventionally been used in the direct dyeing composition or lighting direct dyeing composition, that is, the dye can be chosen from nitrobenzene anthraquinone, azo, neutral, cationic or anionic dyes, naphtoquinone dyes, triarylmethane dyes, xanthene dyes, Arianors and those described in the patent applications WO 95/01772, WO 95/15144 and EP-A-0 714 954. They are used at the concentration between 0.01 and 20 % by weight approximately of the total weight of the composition.

The composition (A) and/or the composition (B) can more particularly contain at least one cationic or amphoteric substantive polymer.

In the present invention, the expression "cationic polymer" denotes all kinds of polymers containing cationic groups and/or groups which can be ionized into cationic groups.

The cationic polymer which can be used in the present invention may be chosen from any of those already known per se as enhancing the cosmetic properties of hair, namely in particular those described in patent application EP-A-337 354 and in French patents FR-2 270 846, 2 383 660, 2 598 611, 2 470 596 and 2 519 863.

The preferred cationic polymer are chosen from those which contain unites containing primary, secondary, tertiary and/or quaternary amine groups which can either form part of the main polymer chain or which can be carried by a lateral substituent that is directly attached thereto.

The cationic polymers used generally have a number-average molecular mass of between 500 and 5 × 10⁶ approximately, and preferably between 10³ and 3 × 10⁶ approximately.

Among the cationic polymers, mention may be made more particularly of polymers of polyamine, polyamino amide and quaternary polyammonium type. These are known products. They are in particular described in French patents No. 2 505 348 or 2 542 997. Among these polymers, mention may be made of:
(1) homopolymers or copolymers derived from acrylic or methacrylic esters or amides and comprising at least one of the units of the following formulae (A), (B), (C) or (D) as follows: 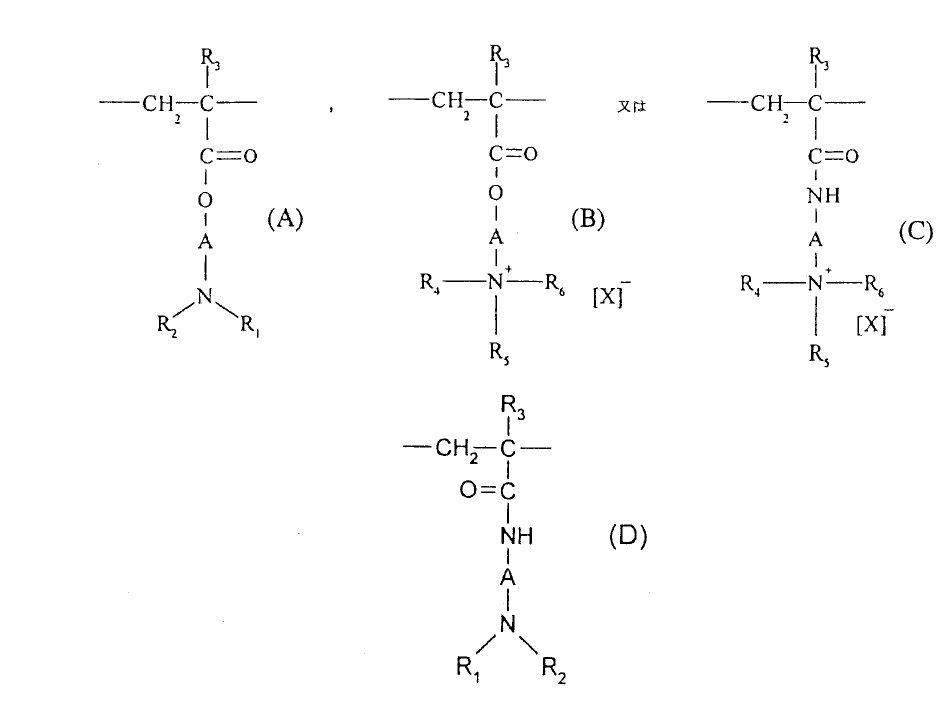 in which :
   R₃, which may be identical or different, denotes a hydrogen atom or a CH₃ radical; A, which may be identical or different, represents a linear or branched alkyl group of 1 to 6 carbon atoms, preferably 2 or 3 carbon atoms, or a hydroxyalkyl group of 1 to 4 carbon atoms;
   R₄, R₅ and R₆ which may be identical or different, represent an alkyl group containing from 1 to 18 carbon atoms or a benzyl radical and preferably an alkyl group containing from 1 to 6 carbon atoms;
   R₁ and R₂, which may be identical or different, represent hydrogen or an alkyl group containing from 1 to 6 carbon atoms and preferably methyl or ethyl;
   X denotes an anion derived from an inorganic or organic acid such as a methosulphate anion or halide such as chloride or bromide.

   The polymer of the family (1) can also contain one or more units derived from comonomers which can be chosen from the family of acrylamides, methacrylamides, diacetone acrylamides, acrylamides and methacrylamides substituted on the nitrogen with lower alkyls (C₁-C₄), acrylic or methacrylic acids or esters thereof, vinyllactams such as vinylpyrrolidone or vinylcaprolactam, and vinyl esters.
   Among the polymers of the family (1), mention may be made of:
   - copolymers of acrylamide and of dimethylaminoethyl methacrylate quaternized with dimethyl sulphate or with a dimethyl halide such as that sold under the name HERCOFLOC by the company HERCULES,
   - copolymers of acrylamide and of methacryloyloxyethyl-trimethylammonium chloride, described, for example, in patent application EP-A-080976 and sold under the name BINA QUAT P 100 by the company CIBA GEIGY,
   - the copolymer of acrylamide and of methacryloyloxyethyltrimethylammonium methosulphate sold under the name RETEN by the company HERCULES,
   - quaternized or non-quaternized vinylpyrrolidone/dialkylaminoalkyl acrylate or methacrylate copolymers, such as the products sold under the name "GAFQUAT" by the company ISP such as, for example, "GAFQUAT 734" or "GAFQUAT 755" or the products known as "COPOLYMER 845, 958 and 937". These polymers are described in detail in French patents 2,077,143 and 2,393,573.
   - the dimethylaminoethyl methacrylate/vinylcaprolactam/vinylpyrrolidone terpolymers, such as the product sold under the name GAFFIX VC 713 by the company ISP,
   - the vinylpyrrolidone/methacrylamidopropyl dimethylamine copolymers sold in particular under the name STYLEZE CC 10 by ISP,
   - and the quaternized vinylpyrrolidone/dimethylaminopropyl methacrylamide copolymers such as the product sold under the name "GAFQUAT HS 100" by the company ISP.
(2) The cellulose ether derivatives containing quaternary ammonium groups, described in French patent No. 1 492 597, and in particular polymers sold under the names "JR" (JR 400, JR 125 and JR 30M) or "LR" (LR 400, or LR 30M) by the company Union Carbide Corporation. These polymers are also defined in the CTFA dictionary as quaternary ammoniums of hydroxyethylcellulose which has reacted with an epoxide substituted with a trimethylammonium group.
(3) Cationic cellulose derivatives such as cellulose copolymers or cellulose derivatives grafted with a water-soluble monomer of quaternary ammonium, and described in particular in the patent US 4 131 576, such as hydroxyalkylcelluloses, for instance hydroxymethyl-, hydroxyethyl- or hydroxypropylcelluloses grafted, in particular, with a methacryloylethyltrimethylammonium, methacrylamidopropyltrimethylammonium or dimethyl-diallylammonium salt.
   The commercial products corresponding to this definition are more particularly the products sold under the names "Celquat L 200" and "Celquat H 100" by the company National Starch.
(4) The cationic polysaccharides described more particularly in the patents US 3 589 578 and 4 031 307 such as guar gums containing cationic trialkylammonium groups. Guar gums modified with a salt (e.g. chloride) of 2,3-epoxypropyltrimethylammonium are used, for example.
   Such products are sold in particular under the trade names JAGUAR C13 S, JAGUAR C 15, JAGUAR C 17 or JAGUAR C162 by the company MEYHALL.
(5) Polymers consisting of piperazinyl unites and of divalent alkylene or hydroxyalkylene radicals containing straight or branched chains, optionally interrupted by oxygen, sulphur or nitrogen atoms or by aromatic or heterocyclic rings, as well as the oxidation and/or quaternization products of these polymers. Such polymers are described, in particular, in French patens 2,162,025 and 2,280,361.
(6) Water soluble polyamino amides prepared in particular by polycondensation of an acidic compound with a polyamine; these polyaminoamides can be crosslinked with an epihalohydrin, a diepoxide, a dianhydride, an unsaturated dianhydride, a bis-unsaturated derivative, a bis-halohydrin, a bis-azetidinium, a bis-haloacyldiamine, a bis-alkyl halide or alternatively with an oligomer resulting from the reaction of a bifunctional compound which is reactive with a bis-halohydrin, a bis-azetidinium, a bis-haloacyldiamine, a bis-alkyl halide, an epihalohydrin, a diepoxide or a bis-unsaturated derivative; the crosslinking agent is used in proportions ranging from 0.025 to 0.35 mol per amine group of the polyamino amide; these polyamino amides can be alkylated or, if they contain one or more tertiary amine functions, they can be quaternized. Such polymers are described, in particular, in French patents 2,252,840 and 2,368,508.
(7) The polyamino amide derivatives resulting from the condensation of polyalkylene polyamines with polycarboxylic acids followed by alkylation with bifunctional agents. Mention may be made, for example of adipic acid/dialkylaminohydroxyalkyldialkylene triamine polymers in which the alkyl radical contains from 1 to 4 carbon atoms and preferably denotes methyl, ethyl or propyl. Such polymers are described in particular in French patent 1,583,363.
   Among these derivatives, mention may be made more particularly of the adipic acid/dimethylaminohydroxypropyl/diethylenetriamine polymers sold under the name "Cartaretine F, F4 or F8" by the company Sandoz.
(8) The polymers obtained by reaction of a polyalkylene polyamine containing two primary amine groups and at least one secondary amine group with a dicarboxylic acid chosen from diglycolic acid and saturated aliphatic dicarboxylic acids having from 3 to 8 carbon atoms. The molar ratio between the plyalkylene polyamine and the dicarboxylic acid is between 0.8:1 and 1.4:1; the polyamino amide resulting therefrom is reacted with epichlorohydrin in a molar ratio of epchlorohydrin relative to the secondary amine group of the polyamino amide of between 0.5:1 and 1.8:1. Such polymers are described in particular in US patent 3,227,615 and 2,961,347.
   Polymers of this type are sold in particular under the name "Hercosett 57" by the company Hercules Inc. or alternatively under the name "PD 170" or "Delsette 101" by the company Hercules in the case of the adipic acid/epoxypropyl/diethylenetriamine copolymer.
(9) Cyclopolymers of alkyldiallylamine or of dialkyl diallylammonium, such as the homopolymers or copolymers containing, as main costituent of the chain, units correxponding to formulae (IV) or (V): 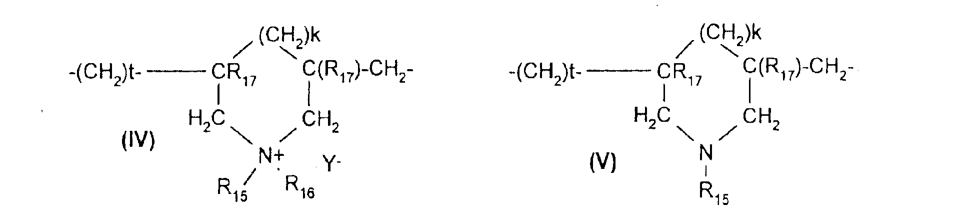 in which formulae k and t are equal to 0 or 1, the sum k + t being equal to 1; R₁₇ denotes a hydrogen atom or a methyl radical; R₁₅ and R₁₆, independently of each other, denote an alkyl group having from 1 to 22 carbon atoms, a hydroxyalkyl group in which the alkyl group preferably has 1 to 5 carbon atoms, or a lower amido(C₁-C₄)alkyl group, or R₁₅ and R₁₆ can denote, together with the nitrogen atom to which they are attached, heterocyclic groups such as piperidinyl or morpholinyl; Y⁻ is an anion such as bromide, chloride, acetate, borate, citrate, tartrate, bisulphate, bisulphite, sulphate or phosphate. These polymers are described in particular in French patent 2,080,759 and in its Certificate of Addition 2,190,406.
   Among the polymers defined above, mention may be made more particularly of the dimethyldiallylammonium chloride homopolymer sold under the name "Merquat 100" by the company Calgon (and its homologues of low weight-average molecular mass) and copolymers of diallyldimethylammonium chloride and of acrylamide sold under the name "Merquat 550".
(10) The quaternary diammonium polymer containing repating units corresponding to the formula: 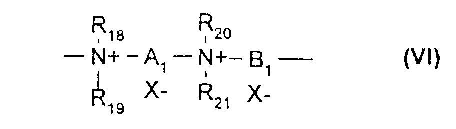 in which formula (VI):
   R₁₈, R₁₉, R₂₀ and R₂₁, identical or different to each other, represent aliphatic, alicyclic or arylaliphatic radicals containing from 1 to 20 carbon atoms or lower hydroxyalkylaliphatic radicals, or alternatively R₁₈, R₁₉, R₂₀ and R₂₁, together or separately, constitute, with the nitrogen atoms to which they are attached, heterocycles optionally containing a second heteroatom other than nitrogen, or alternatively R₁₈, R₁₉, R₂₀ and R₂₁ represent a linear or branched C₁-C₆ alkyl radical substituted with a nitrile, ester, acyl or amide group or a group -CO-O-R₂₂-D or -CO-NH-R₂₂-D where R₂₂ is an alkylene and D is a quaternary ammonium group;
   A₁ and B₁ represent polymethylene groups containing from 2 to 20 carbon atoms which may be linear or branched, saturated or unsaturated, and which may contain, linked to or intercalated in the main chain, one or more aromatic rings or one or more oxygen or sulphur atoms or sulphoxide, sulphone, disulphide, amino, alkylamino, hydroxyl, quaternary ammonium, ureido, amide or ester groups, and
   X⁻ denotes an anion derived from an inorganic or organic acid;
   A₁, R₁₈ and R₂₀ can form, with the two nitrogen atoms to which they are attached, a piperazine ring; in addition, if A₁ denotes a linear or branched, saturated or unsaturated alkylene or hydroxyalkylene radical, B₁ can also denote a group

      - (CH₂)n-CO-D-OC-(CH₂)ₙ-

      wherein D denotes:
      a) a glycol residue of formula : -O-Z-O-, where Z denotes a linear or branched hydrocarbon radical or a group corresponding to one of the following formulae;

         -(CH₂-CH₂-O)ₓ-CH₂-CH₂-

         -[CH₂-CH(CH₃)-O]_{y}-CH₂-CH(CH₃)-

         where x and y denote an integer from 1 to 4 representing a defined and unique degree of polymerization or an number from 1 to 4 representing an average degree of polymerization;
      b) a bis-secondary diamine residue such as a piperazine derivative;
      c) a bis-primary diamine residue of formula: -NH-Y-NH-, wherein Y denotes a linear or branched hydrocarbon radical, or alternatively the divalent radical

         -CH₂-CH₂-S-S-CH₂-CH₂-;
      d) a ureylene group of formula: -NH-CO-NH-.

   Preferably, X⁻ is an anion such as chloride or bromide.
   These polymers generally have a number average molecular mass of between 1000 and 100,000.
   Polymers of this type are describe in particular in French Patents 2,320,330, 2,270,846, 2,316,271, 2,336,434 and 2,413,907 and US patents 2,273,780, 2,375,853, 2,388,614, 2,454,547, 3,206,462, 2,261,002, 2,271,378, 3,874,870, 4,001,432, 3,929,990, 3,966,904, 4,005,193, 4,025,617, 4,025,627, 4,025,653, 4,026,945 and 4,027,020.
   It is more particularly possible to use the polymers consisting of repeating units corresponding to the formula: 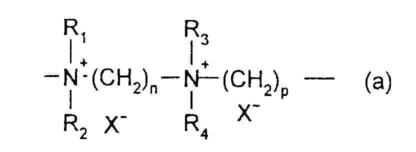 in which R₁, R₂, R₃ and R₄, which may be identical or different, denote an alkyl or hydroxyalkyl radical containing from 1 to 4 carbon atoms approximately, n and p are integers ranging from 2 to 20 approximately and X⁻ is an anion derived from an inorganic or organic acid.
(11) Quaternary polyammonium polymers consisting of units of formula (VII): 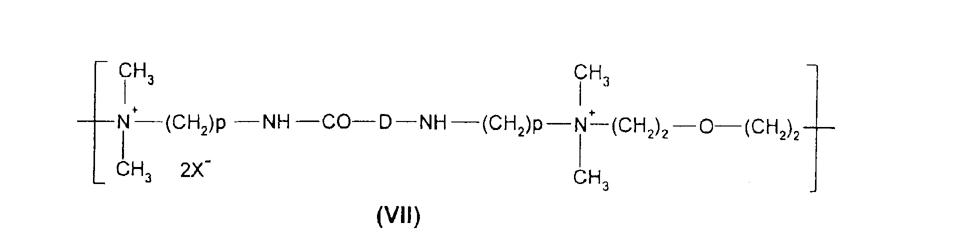 in which:
   p denotes an integer varying from 1 to 6 approximately,
   D may be nil or may represent a -(CH₂)ᵣ-CO- group wherein r denotes a number equal to 4 or 7, and
   X⁻ is an anion derived from an inorganic or organic acid.

   The cationic polymers having the units of formula (VII) are for example described in the patent application EP-A-122 324 and may be prepared according to the procedures described in the U.S. Patent No. 4 157 388, 4 390 689, 4 702 906 and 4 719 282.
   Among these polymers, preferred are those having the molecular weight measured by NMR of Carbon 13 less than 100000, and in the formula, p is equal to 3, and
   a) D represents a -(CH₂)₄-CO- group, X denotes a chlorine atom, the molecular weight measured by NMR of Carbon 13 (NMR¹³C) being approximately 5600; this type of polymer is proposed by the company MIRANOL under the name MIRAPOL-AD1,
   b) D represents a -(CH₂)₇-CO- group, X denotes a chlorine atom, the molecular weight measured by NMR of Carbon 13 (NMR¹³C) being approximately 8100; this type of polymer is proposed by the company MIRANOL under the name MIRAPOL-AZ1,
   c) D represents a zero value, X denotes a chlorine atom, the molecular weight measured by NMR of Carbon 13 (NMR¹³C) being approximately 25500; this type of polymer is proposed by the company MIRANOL under the name MIRAPOL-A15,
   d) a "Block Copolymer" formed of the units corresponding to the polymers described in paragraphs a) and c), proposed by the company MIRANOL under the names NIRAPOL-9, (molecular weight NMR¹³C, approximately 7800) MIRAPOL-175, (molecular weight NMR¹³C, approximately 8000) MIRAPOL-95, (molecular weight NMR¹³C, approximately 12500).

   More particularly again, according to the invention, preferred is the polymer of the unit of formula (VII) in which p is equal to 3, D denotes a zero value, X denotes a chlorine atom, the molecular weight measured by NMR of Carbon 13, (NMR13C) being approximately 25500.
(12) Quaternary polymers of vinylpyrrolidone and of vinylimidazole, such as, for example, the products sold under the names Luviquat FC 905, FC 550 and FC 370 by the company BASF.
(13) Polyamines such as Polyquart H sold by HENKEL under the reference name "POLYETHYLENE GLYCOL (15) TALLOW POLYAMINE" in the CTFA dictionary.
(14) Crosslinked polymers of methacryloyloxy(C₁-C₄)alkyltri(C₁-C₄)alkylammonium salts such as the polymers obtained by homopolymerization of dimethylaminoethyl methacrylate quaternized with methyl chloride, or by copolymerization of acrylamide with dimethylaminoethyl methacrylate quaternized with methyl chloride, the homo- or copolymerization being followed by crosslinking with a compound containing olefinic unsaturation, in particular methylenebisacrylamide. A crosslinked acrylamide/methacryloyloxyethyltrimethylammonium chloride copolymer (20/80 by weight) in the form of a dispersion containing 50% by weight of the said copolymer in mineral oil can be used more particularly. This dispersion is sold under the name "SALCARE® SC 92" by the company ALLIED COLLOIDS. A crosslinked methacryloyloxyethyltrimethylammonium chloride homopolymer containing about 50% by weight of the homopolymer in mineral oil or in a liquid ester can also be used. These dispersions are sold under the names "SALCARE® SC 95" and "SALCARE® SC 96" by the company ALLIED COLLOIDS.

Other cationic polymers which can be used in the context of the invention are polyalkyleneimines, in particular polyethyleneimines, polymers containing vinylpyridine or vinylpyridinium unites, condensates of polyamines and of epichlorohydrin, quaternary polyureylenes and chitin derivatives.

Among all the cationic polymers which can be used in the context of the present invention, polymers of the family (1), (9), (10), (11) and (14) are preferred, and more preferably, polymers of the formula (W) and (U) below are preferable: 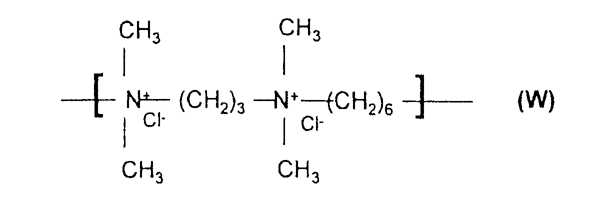 and in particular those of which the molecular weight, determined by gel chromatography, is between 9500 and 9900; 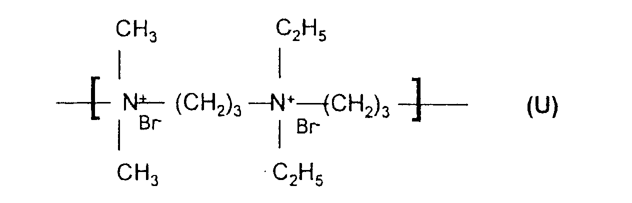 and in particular those of which the molecular weight, determined by gel chromatography, is approximately 1200.

Concentration of the cationic polymer in the composition according to the present invention is 0.01 to 10% by weight, preferably 0.05 to 5% by weight, more preferably 0.1 to 3% by weight of the total weight of the composition.

The amphoteric polymers which can be used in the present invention can be selected from the polymers containing K and M units distributed statistically in the polymer chain, where K denotes a unit derived from a monomer containing at least one basic nitrogen atom and M denotes a unit derived from an acid monomer containing one or more carboxylic or sulphonic groups, or alternatively K and M can denote groups derived from zwitterionic monomers of carboxybetaines or of sulphobetaines; K and M may also denote a cationic polymer chain containing primary, secondary, tertiary or quaternary amine groups, in which at least one of the amine groups carries a carboxylic or sulphonic group linked via a hydrocarbon radical or alternatively K and M form part of a chain of a polymer with an α,β-dicarboxylic ethylene unit in which one of the carboxylic groups has been caused to react with a polyamine containing one or more primary or secondary amine groups.

The film forming amphoteric polymers defined above are more preferably selected from the following polymers:
(1) The polymers resulting from the copolymerization of a monomer derived from a vinyl compound carrying a carboxylic group such as more particularly acrylic acid, methacrylic acid, maleic acid, alpha-chloroacrylic acid, and of a basic monomer derived from a substituted vinyl compound containing at least one basic atom such as more particularly dialkylaminoalkyl methacrylate and acrylate, dialkylaminoalkylmethacrylamide and acrylamide. Such compounds are described in U.S. Patent No. 3 836 537. Copolymer of the sodium acrylate/acrylamidopropyltrimethylammonium chloride sold under the name of "POLYQUART KE 3033" by the company HENKEL can also be cited.
   The vinyl compound can also be a salt of dialkyldiallylammonium such as diethyldiallylammonium chloride. The copolymers of acrylic acid and the latter monomer are proposed under the name "MERQUAT 280", "MERQUAT 295" and "MERQUAT PLUS 3330" by the company CALGON.
(2) The polymers containing units which are derived from:
   a) at least one monomer chosen from acrylamides or methacrylamides substituted on the nitrogen by an alkyl radical,
   b) at least one acidic comonomer containing one or more reactive carboxylic groups, and
   c) at least one basic comonomer such as esters with primary, secondary,
   tertiary and quaternary amine substituents of acrylic and methacrylic acids and the product of quaternization of dimethylaminoethyl methacrylate with dimethyl or diethyl sulphate.
   The N-substituted acrylamides or methacrylamides most particularly preferred according to the invention are groups whose alkyl radicals contain from 2 to 12 carbon atoms and more particularly N-ethylacrylamide, N-tert-butylacrylamide,
   N-tert-octylacrylamide, N-octylacrylamide, N-decylacrylamide, N-dodecylacrylamide as well as the corresponding methacrylamides.
   The acidic comonomers are chosen more particularly from acrylic, methacrylic, crotonic, itaconic, maleic and fumaric acids as well as the alkyl monoesters having 1 to 4 carbon atoms of maleic or fumaric anhydrides or acids.
   The preferred basic comonomers are methacrylates of aminoethyl, butylaminoethyl, N,N'-dimethylaminoethyl, N-tert-butylaminoethyl.
   Particularly used are the copolymers of which the CTFA name (4^{th} edition, 1991) are Octylacrylamide/acrylates/butylaminoethylmethacrylate copolymer such as the products sold under the name AMPHOMER or LOVOCRYL 47 by the company NATIONAL STARCH.
(3) The partially or completely alkylated and crosslinked polyaminoamides derived from polyaminoamides of general formula:

   ⁅CO-R₂₇-CO-Z⁆ (VIII)

   in which R₂₇ represents a divalent radical derived from a saturated dicarboxylic acid, a mono- or dicarbocylic aliphatic acid with ethylenic double bond, an ester of a lower alkanol having 1 to 6 carbon atoms of these acids or a radical which is derived from the addition of any one of the said acids with a bis-primary or bis-secondary amine, and Z denotes a radical of a bis-primary, mono- or bis-secondary polyalkylene-polyamine and preferably represents:
   a) in the proportions of 60 to 100 mol %, the radical 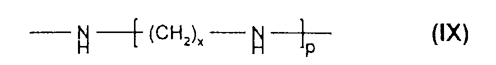 wherein x=2 and p=2 or 3, or alternatively x=3 and p=2,
      this radical being derived from the diethylenetriamine, triethylenetetraamine or dipropylenetriamine;
   b) in the proportions of 0 to 40 mol %, the radical (IX) above in which x=2 and p=1 and which is derived from ethylenediamine, or the radical which is derived from piperazine: 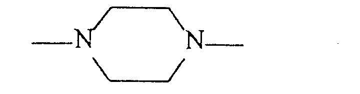
   c) in the proportions of 0 to 20 mol %, the radical -NH-(CH₂)₆-NH- which is derived from hexamethylenediamine, these polyamino amines being crosslinked by adding a bifunctional crosslinking agent chosen from the epihalohydrins, diepoxides, dianhydrides, bis-unsaturated derivatives, by means of 0.025 to 0.35 mol of crosslinking agent per amine group of the polyamino amide and alkylated by the action of acrylic acid, chloroacetic acid or of an alkanesultone or of their salts.
   The saturated carboxylic acids are preferably chosen from the acids having 6 to 10 carbon atoms such as adipic, 2,2,4-trimethyladipic and 2,4,4-trimethyladipic acid, terephthalic acid, the acids with ethylene double bond such as for example acrylic, methacrylic and itaconic acids.
   The alkanesultones used in the alkylation are preferably propane or butanesultone, the salts of the alkylating agents are preferably the sodium or potassium salts.
(4) The polymers containing zwitterionic units of formula: 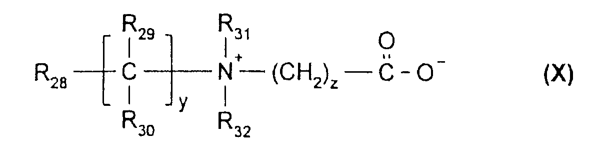 in which R₂₈ denotes a polymerizable unsaturated group such as an acrylate, methacrylate, acrylamide or methacrylamide group, y and z represent an integer from 1 to 3, R₂₉ and R₃₀ represent a hydrogen atom, methyl, ethyl or propyl, R₃₁ and R₃₂ represent a hydrogen atom or an alkyl radical such that the sum of the carbon atoms in R₃₁ and R₃₂ does not exceed 10.
   The polymers comprising such units may also comprise units derived from nonzwitterionic monomers such as dimethyl or diethylaminoethyl acrylate or methacrylate or alkyl acrylates or methacrylates, acrylamides or methacrylamides or vinyl acetate.
   By way of example, there may be mentioned the copolymer of methyl methacrylate/methyl dimethylcarboxymethylammonioethyl methacrylate such as the product sold under the name DIAFORMER Z301 by the company SANDOZ.
(5) The polymers derived from chitosan containing monomeric units corresponding to the following formulae (D), (E) and (F): 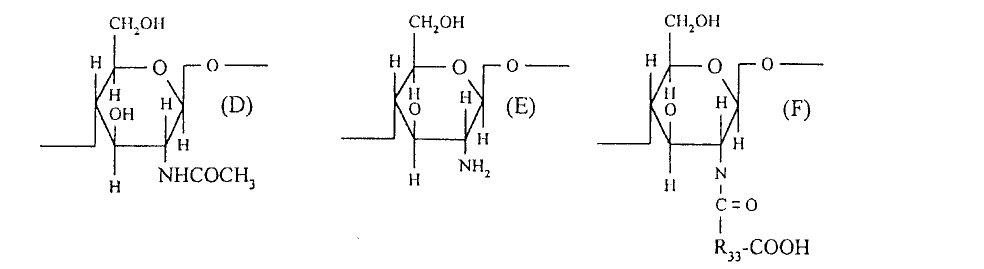 the unit D being present in proportions of between 0 and 30%, the unit E in proportions of between 5 and 50% and the unit F in proportions of between 30 and 90%, it being understood that in this unit F, R₃₃ represent a radical of formula: 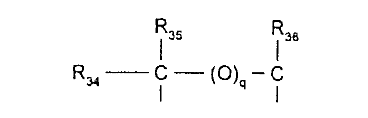 in which if q=0, R₃₄, R₃₅ and R₃₆, which are identical or different, each represent a hydrogen atom, a methyl, hydroxyl, acetoxy or amino residue, a monoalkylamine residue or a dialkylamine residue optionally interrupted by one or more nitrogen atoms and/or optionally substituted with one or more amine, hydroxyl, carboxyl, alkylthio or sulphonic groups, or an alkylthio residue whose alkyl group carries an amino residue, at least one of the R₃₄, R₃₅ and R₃₆ radicals being in this case a hydrogen atom; or if q=1, R₃₄, R₃₅ and R₃₆ each represent a hydrogen atom, as well as the salts formed by these compounds with bases or acids.
(6) The polymers derived from the N-carboxyalkylation of chitosan such as N-carboxymethyl chitosan or N-carboxybutyl chitosan sold under the name "EVALSAN" by the company JAN DEKKER.
(7) The polymers corresponding to the general formula (XI) are described for example in French Patent 1 400366: 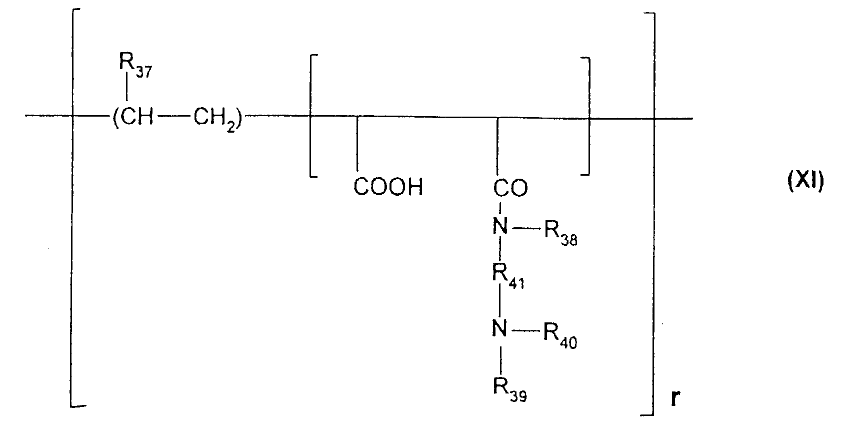 wherein R₃₇ represents a hydrogen atom, a CH₃O, CH₃CH₂O, phenyl radical, R₃₈ denotes hydrogen or a lower alkyl radical such as methyl or ethyl, R₃₉ denotes hydrogen or a lower alkyl radical such as methyl or ethyl, R₄₀ denotes a lower alkyl radical such as methyl or ethyl or a radical corresponding to the formula: -R₄₁-N(R₃₉)₂, R₄₁ representing -CH₂-CH₂-, -CH₂-CH₂-CH₂-, -CH₂-CH(CH₃)-, R₃₉ having the above-described meaning,
   as well as the higher homologues of these radicals and containing up to 6 carbon atoms.
(8) Amphoteric polymers of the -D-X-D-X- type chosen from:
   a) the polymers obtained by the action of chloroacetic acid or sodium chloroacetate on the compounds containing at least one unit of formula:

      -D-X-D-X-D- (XII)

      wherein D denotes a radical 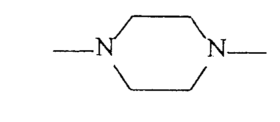 and X denotes the symbol E or E', E or E', which are identical or different, denote a bivalent radical which is an alkylene radical with a linear or branched chain containing up to 7 carbon atoms in the principal chain which is unsubstituted or substituted with hydroxyl groups and which may contain, in addition, oxygen, nitrogen or sulphur atoms, 1 to 3 aromatic and/or heterocyclic rings; the oxygen, nitrogen and sulphur atoms being present in the form of ether, thioether, sulphoxide, sulphone, sulphonium, alkylamine or alkenylamine groups, or hydroxyl, benzylamine, amine oxide, quaternary ammonium, amide, imide, alcohol, ester and/or urethane groups.
   b) The polymers of formula:

      -D-X-D-X- (XIII)

      where D denotes a radical 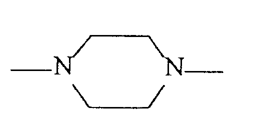 and X denotes the symbol E or E' and, at least once, E'; E having the meaning indicated above and E' is a bivalent radical which is an alkylene radical with a linear or branched chain having up to 7 carbon atoms in the principal chain, which is unsubstituted or substituted with one or more hydroxyl radicals and containing one or more nitrogen atoms, the nitrogen atom being substituted with an alkyl chain optionally interrupted by an oxygen atom and necessarily containing one or more carboxyl functional groups or one or more hydroxyl functional groups and betainized by reaction with chloroacetic acid or sodium chloroacetate.
(9) The copolymers (C₁-C₅)alkyl vinyl ether/maleic anhydride partially modified by semiamidation with an N,N-dialkylaminoalkylamine such as N,N-dimethylaminopropylamine or by semiesterification with an N,N-dialkanolamine. These copolymers may also contain other vinyl comonomers such as vinylcaprolactam.

The amphoteric polymers particularly preferred according to the invention are those belonging to the family (1).

According to the invention, the cationic or amphoteric polymer or polymers may represent from 0.01 % to 10 % by weight, preferrably 0.05 % to 5 % by weight, further preferably, 0.1 % to 3 % by weight of the total weight of the composition.

The composition according to the invention contain preferably one or more surfactants. The surfactant or surfactants may be chosen independently, solely or as a mixture, from the anionic, amphoteric, nonionic, zwitterionic and cationic surfactants.

The surfactants which may be used for carrying out the present invention are especially the following:

### (i) Anionic surfactant(s):

By way of example of anionic surfactants that can be employed, solely or as mixtures, in the context of the present invention, there may be mentioned especially (non limiting listing) the salts (in particular alkali metal, especially sodium salts, ammonium salts, amine salts, amino alcohol salts or magnesium salts) of the following compounds: alkyl sulphates, alkyl ether sulphates, alkylamido ether sulphates, alkylaryl-polyether sulpahtes, monoglyceride sulphates, alkyl sulphonates, alkyl phosphates, alkylamidesulphonates, alkyl aryl sulphonates, α-olefin-sulphonates, paraffin-sulphonates, alkyl(C₆-C₂₄) sulphosuccinates, alkyl(C₆-C₂₄) ether sulphosuccinates, alkyl(C₆-C₂₄)amidesulphosuccinates, alkyl(C₆-C₂₄) sulphoacetates, acyl(C₆-C₂₄) sarcosinates, acyl(C₆-C₂₄) glutamates. What can also be employed are alkyl(C₆-C₂₄)polyglycoside carboxylic esters such as alkylglucoside citrates, alkylpolyglycoside tartrate and alkylpolyglycoside sufosuccinates, alkylsulfosuccinamates; acylisethionates and N-acyltaurates, the alkyl or acyl radical of all these different compounds preferably containing from 12 to 20 carbon atoms, and the aryl radical preferably denoting a phenyl or benzyl group. Among the anionic surfactants which are further usable, there may also be mentioned the salts of fatty acids such as the salts of oleic, ricinoleic, palmitic and stearic acids, the acids of copra oil or of hydrogenetaed copra oil; and acyl lactylates in which the acyl radical contains 8 to 20 carbon atoms. It is also possible to employ alkyl-D-galactosideuronic acids and salts thereof, polyoxyalkylenated carboxylic (C₆-C₂₄)alkyl ether acid, the polyoxyalkylenated carboxylic (C₆-C₂₄)alkylaryl ether acids, the polyoxyalkylenated carboxylic (C₆-C₂₄)alkyl amidoether acids and their salts, in particular those containing from 2 to 50 ethylene oxide groups and mixtures thereof.

### (ii) Nonionic surfactant(s):

The nonionic surfactants themselves are also compounds which are well known per se (in this respect, see especially the "Handbook of Surfactants" by M.R. Porter, published by Blackie & Son (Glasgow and London), 1991, pp. 116-178) and, in the context of the present invention, their nature is not of critical importance. They can thus be chosen especially from (nonlimiting listing) alcohols, alpha-diols, or alkylphenols. The copolymers of ethylene oxide and propylene oxide and the condensates of ethylene oxide and propylene oxide with fatty alcohols; the polyethoxylated fatty amides preferably containing from 2 to 30 mol of ethylene oxide, the polyglycerolated fatty amides on average containing 1 to 5 glycerol groups and in particular 1.5 to 4; the oxyethylenated fatty acid esters of sorbitan containing from 2 to 30 mol of ethylene oxide; the fatty acid esters of sucrose, the fatty acid esters of polyethylene glycol, alkylpolyglycosides, the N-alkylglucamine derivatives, amine oxides such as the oxides of (C₁₀-C₁₄) alkylamines or the N-acylaminopropylmorpholine oxides may also be mentioned. It will be noted that alkylpolyglycosides constitute nonionic surfactants which are particularly well suited within the context of the present invention.

### (iii) Amphoteric or zwitterionic surfactant(s):

The amophoteric or zwitterionic surfactants, of which nature is not a critical feature in the context of the present invention, can be, in particular (non-limiting listing), aliphatic secondary or tertiary amine derivatives in which the aliphatic radical is a linear or branched chain containing 8 to 18 carbon atoms and containing at least one water-solubilizing anionic group (for example carboxylate, sulphonate, sulphate, phosphate or phosphonate); mention may also be made of (C₈-C₂₀)alkylbetaines, sulphobetaines, (C₈-C₂₀)alkylamido(C₁-C₆)alkylbetaines or (C₈-C₂₀)alkylamido(C₁-C₆)alkylsulphobetaines.

Among the amine derivatives, mention may be made of the products sold under the name MIRANOL, as described in the patents US-2 528 378 and US-2 781 354 and classified in the CTFA dictionary, 3^{rd} edition, 1982, under the names Amphocarboxyglycinates and Amphocarboxypropionates, having the respective structures:

R₂-CONHCH₂CH₂-N(R₃)(R₄)(CH₂COO⁻)

wherein: R₂ denotes an alkyl radical derived from an acid R₂-COOH present in hydrolysed coconut oil, a heptyl, nonyl or undecyl radical, R3 denotes a beta hydroxyethyl group and R4 denotes a carboxymethyl group; and

R₂,-CONHCH₂CH₂-N(B)(C)

wherein:
B represents -CH₂CH₂OX', C represents -(CH₂)z-Y', with z=1 or 2,
X' denotes the -CH₂CH₂-COOH group or a hydrogen atom,
Y' denotes -COOH or the -CH₂-CHOH-SO₃H radical,
R_{2'} denotes an alkyl radical of an acid R₉ -COOH present in coconut oil or in hydrolysed linseed oil, an alkyl radical, in particular a C₇, C₉, C₁₁ or C₁₃ alkyl radical, a C₁₇ alkyl radical and its iso form, or an unsaturated C₁₇ radical.

These compounds are classified in the dictionary CTFA, 5^{th} edition, 1993 under the denomination Disodium Cocoamphodiacetate, Disodium Lauroamphodiacetate, Disodium Caprylamphodiacetate, Disodium Capryloamphodiacetate, Disodium Cocoamphodipropionate, Disodium Lauroamphodipropionate, Disodium Caprylamphodipropionate, Disodium Capryloamphodipropionate, Lauroamphodipropionic acid, Cocoamphodipropionic acid. By way of example, mention may be made of the cocoamphodiacetate commercialized under the trade name concentrated MIRANOL® C2M by the company PHODIA CHIMIE.

### (iv) Cationic surfactants:

Among the cationic surfactants, it is possible to particularly cite (non limiting list) salts of optionally polyoxyalkylenated primary, secondary or tertiary fatty amine; quaternary ammonium salts such as tetraalkylammonium, alkylamidoalkyltrialkylammonium, trialkylbenzylammonium, trialkylhydroxyalkylammonium or alkylpyridinium chlorides or bromides; imidazoline derivatives; or amine oxides of cationic nature.

The quantity of surfactants present in the composition according to the present invention may vary between 0.01 and 40 % and preferably between 0.5 and 30% of the total weight of the composition.

The composition according to the present invention may eventually contain other agents for adjustment of rheology, such as the cellulose thickeners (hydroxyethylcellulose, hydroxypropylcellulose, carboxymethylcellulose ..), guar gum and its derivatives (hydroxypropylguar ..), gum of microbial origin (xanthan gum, scleroglucan gum ..), synthetic thickeners such as crosslinked homopolymers of acrylic acid or acrylamidopropanesulfonic acid and ionic or nonionic associative polymers such as the polymers commercialized under the trade name PEMULEN TR1 or TR2 by the company GOODRICH, SALCARE SC90 by the company ALLIED COLLOIDS and ACULYN 22, 28, 33, 44 or 46 by the company ROHM and HAAS, and ELFACOS T210 and T212 by the company AKZO.

These thickeners may represent from 0.01 to 10 % by weight of the total weight of the composition.

The medium of the composition appropriate for dyeing is preferably an aqueous medium constituted by water, which may advantageously contain the cosmetically acceptable organic solvents, which is more particularly alcohols such as ethyl alcohol, isopropyl alcohol, benzyl alcohol, and phenylethyl alcohol, or glycols or ethers of glycol such as, for example, monomethyl, monoethyl and monobutyl ethers of ethyleneglycol, propyleneglycol or their ethers such as, for example, monomethylether of propyleneglycol, butyleneglycol, dipropyleneglycol as well as alkylethers of diethyleneglycol such as, for example, monoethylether or monobutylether of diethyleneglycol, at concentrations between approximately 0.5 and 20 % and, preferably, between approximately 2 and 10 % by weight of the total weight of the composition.

The composition (A) may further contain an effective quantity of other agents for example which are already known for oxidation coloration, such as various ordinary adjuvants such as sequesterizers such as EDTA and etidronic acid, UV filters, waxes, volatile or nonvolatile, cyclic or non-cyclic, linear or branched, organomodified (especially by amine groups) silicones, preservatives, ceramides, pseudoceramides, vegetable, mineral or synthetic oils, vitamines or provitamines such as panthenol, opacifiers, etc..

The composition can also contain the reducing agents or antioxidants. They may be chosen particularly among sodium sulphite, thioglycolic acid, thiolactic acid, sodium bisulphite, dehydroascorbic acid, hydroquinone, 2-methylhydroquinone, tert-butylhydroquinone and homogentisic acid, and they may be present in the quantity of approximately 0.05 to 1.5 % by weight of the total weight of the composition.

Needless to say, a person skilled in the art will take care to select this or those optionally complementary compounds, such that the advantageous properties intrinsically associated with the dye composition according to the invention are not, or are not substantially, adversely affected by the additions envisaged.

In the composition ready for use in the composition (B), the oxidizing agent is chosen preferably among urea peroxide, alkali metal bromates or ferricyanides, persalts such as perborates and persulfates. The use of hydrogen peroxide is particularly preferable. This oxidizing agent is advantageously constituted by an oxygenated aqueous solution of which the titre may vary more particularly approximately 1 to 40 in volume, more preferably approximately 5 to 40.
As an oxidizing agent, one or more oxidation-reduction enzymes such as laccases, peroxydases and 2-electron oxydoreductases (such as uricase), if necessary in the presence of their respective donner or cofacter, may be used.

The pH of the composition ready for use on the keratin fibers [composition resulting from mixing the dye composition (A) and the oxidizing composition (B)], is generally between the value 4 and 11. It is preferable to be within 6 and 10, and may be adjusted to the desired value by means of acidifying or basifying agents well known in the art of dyeing keratin fibers.

Among the basifying agents which may be mentioned, for example, are aqueous ammonia, alkaline carbonates, alkanolamines such as mono-, di- and triethanolamines and derivatives thereof, hydroxyalkylamines and oxyethylenated and/or oxypropylenated ethylenediamines, sodium or potassium hydroxide and the compounds of formula (XIV) below: 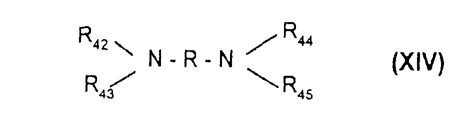 in which R is a propylene residue optionally substituted with a hydroxyl group or a C₁-C₄ alkyl; R₄₂, R₄₃, R₄₄ and R₄₅, which may be identical or different, represent a hydrogen atom or a C₁-C₄ alkyl or C₁-C₄ hydroxyalkyl radical.

The acidifying agents are classically, by way of example, organic or inorganic acids such as hydrochloric acid, orthophosphoric acid, carboxylic acids such as tartaric acid, citric acid, lactic acid or sulphonic acids.

Examples illustrating the invention will now be described, in order to describe the invention without the limiting nature.

### Example 1:

We prepared the dye composition according to the invention, as follows:

| Composition A' | |
|---|---|
| Oxyethylenated fatty alcohols | 21 |
| Lauric acid | 3 |
| Cetylstearyl alcohol | 11.5 |
| Polyacrylic acid | 0.4 |
| Silica | 1.2 |
| Opacifying agent | 2 |
| Propylene glycol | 10 |
| Cationic polymer of formula (W) in a 60 % aqueous solution | 5 |
| Merquat 280 | 3.7 |
| Sequestering agent | qs |
| Reducing agent | qs |
| Ammonia (20 % NH₃) | 11 |
| Oxidation dye | qs |
| Water qsp | 100 |

| Composition A" | |
|---|---|
| Ceteareth 60 myristyl glycol (ELFACOS GT 282 S of company AKZO) | 6.0 g |
| Diisopropyl adipate | 50 g |
| Benzoate of C₁₂-C₁₅ alcohol | 10 g |
| Preservatives | qs |
| Water qsp | 100 |

At the moment of use, 10g of composition A' was mixed with lg of composition A" and 15g of oxygenated water solution at 20 volumes.
A thick and stable composition was obtained. Then, the composition obtained was applied to locks of permed hair containing 90 % white hairs. After pausing 30 minutes, the locks were rinsed, then washed with shampoo, rinsed again and then dried. The hair was dyed to a natural brown color.

### Example 2:

We prepared the dye composition according to the invention, as follows:

| | |
|---|---|
| ELFACOS GT 282 S | 1 |
| Oxyethylenated fatty alcohols | 21 |
| Lauric acid | 3 |
| Cetylstearyl alcohol | 11.5 |
| Polyacrylic acid | 0.4 |
| Silica | 1.2 |
| Opacifying agent | 2 |
| Propylene glycol | 10 |
| Cationic polymer of formula (W) in a 60 % aqueous solution | 5 |
| Merquat 280 | 3.7 |
| Sequestrating agent | qs |
| Reducing agent | qs |
| Ammonia (20% NH₃) | 11 |
| Oxidation dye | qs |
| Water qsp | 100 |

At the moment of use, this composition was mixed with oxygenated water solution at 20 volumes. A thick and stable composition was obtained. Then the composition obtained was applied to locks of permed hair containing 90 % white hairs. After 30 minutes of pause, the locks of hair were rinsed, then washed with shampoo, rinsed again and then dried.
The hair was dyed to a natural brown color.

## Claims

1. A composition for dyeing keratin fibers, particularly for human keratin fibers such as hair, comprising, in a medium suitable for dyeing, at least one dye and at least one nonionic compound of the formula R-(OCH₂CH₂)ₙ,-OR', wherein R is a C₁₀-C₃₀ alkyl radical, R' is a C₁₀-C₃₀ alkyl radical which may be substituted with a hydroxyl radical, and n is from 1 to approximately 100.

2. A composition according to claim 1, wherein R is a C₁₆-C₁₈ alkyl radical, R' is a C₁₆-C₁₈ alkyl radical which may be substituted by a hydroxyl radical, and n is equal to or larger than 20.

3. A composition according to claim 1 or 2, wherein R is a C₁₆-C₁₈ alkyl radical and R' is an alkyl radical C₁₄OH.

4. A composition according to claim 3, wherein n is 60.

5. A composition according to claim 1, wherein R and R' are each a C₁₆-C₁₈ alkyl radical and n is 4.

6. A composition according to any preceding claim, wherein the nonionic compound is present at the concentration of from 0.05 to 10% by weight with respect to the total weight of the composition.

7. A composition according to claim 6, wherein the nonionic compound is present at the concentration of from 0.1 to 5% by weight with respect to the total weight of the composition.

8. A composition according to any preceding claim, wherein the dye is an oxidation dye.

9. A composition according to any of claims 1 to 7, wherein the dye is a direct dye.

10. A composition according to any preceding claim, wherein the composition is a composition ready to use for dyeing keratin fibers, constituted with a composition A comprising, in a medium appropriate for dyeing, at least one oxidation dye, and/or a direct dye, and a composition B comprising an oxidizing agent, the nonionic compound as defined in any of claims 1 to 5 being contained in the composition A or in the composition B, or in both of the compositions A and B.

11. A composition according to claim 8 or 10, wherein the oxidation dye is an oxidation base chosen from paraphenylenediamine, double bases, ortho- or para-aminophenols and heterocyclic bases and addition salts of these compounds with an acid.

12. A composition according to claim 8 or 10, wherein the oxidation dye is a coupler chosen from meta-phenylenediamines, meta-aminophenols, meta-diphenols, the mono- or poly-hydroxylated derivatives of naphthalene, sesamol and its derivatives and heterocyclic compounds such as indole couplers, indoline couplers, pyridine couplers, and their addition salts with an acid.

13. A composition according to any of claims 8 or 10 to 12, which comprises at least one oxidation base and at least one coupler.

14. A composition according to claim 11 or 12, wherein the addition salts with an acid are chosen from hydrochlorides, hydrobromides, sulphates and tartrates, lactates and acetates.

15. A composition according to any of claims 11, 13 or 14, wherein the oxidation base is present at a concentration of from 0.0005 to 12% by weight of the total weight of the composition A.

16. A composition according to any of claims 12, 13 or 14, wherein the coupler is present at a concentration of from 0.0001 to 10% by weight of the total weight of the composition A.

17. A composition according to any of claims 10 to 16, wherein the oxidising agent of composition B is chosen from hydrogen peroxide, urea peroxide, alkali metal bromates or ferricyanides, organic peroxides, persalts or oxidation-reduction enzymes.

18. A composition according to claim 17, wherein the oxidising agent is an oxygenated water solution of which the titre varies from 1 to 40 volumes.

19. A composition according to claims 10 to 18, wherein the direct dye is chosen from nitrobenzene, anthraquinone, azo, neutral, cationic or anionic dyes, naphtoquinone dyes, triarylmethane dyes, and xanthene dyes.

20. A composition according to any preceding claim, which comprises from 0.01 to 40% by weight of at least one nonionic, anionic, cationic, amphoteric or zwitterionic surfactant.

21. A composition according to any preceding claim, which comprises from 0.01 to 10% by weight of at least one cationic or amphoteric polymer.

22. A composition according to claim 21, wherein the cationic polymer is constituted by repeating units of the formulae (W) or (U) as follows:

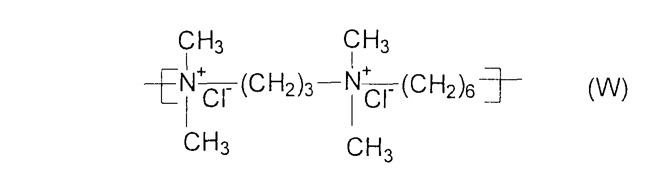

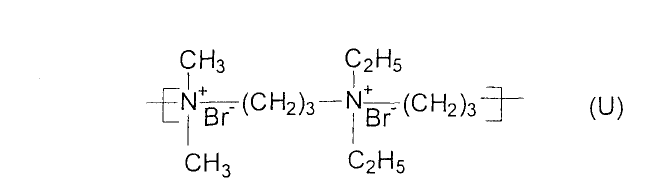

23. A composition according to any preceding claim, wherein the medium appropriate for dyeing is an aqueous medium constituted by water and optionally organic solvents at a concentration of from 0.5 to 20%.

24. A process for dyeing keratin fibers, and in particular human keratin fibers such as hair, which process comprises applying to the dry or wet keratin fibers at least one composition containing, in a medium appropriate for dyeing, at least one direct dye and at least one nonionic compound as defined in any of claims 1 to 5, optionally leaving the composition to act on the fibers for a resting time of from 3 to 60 minutes, rinsing the fibers, optionally washing and rinsing the fibers, and then drying the fibers.

25. A process for dyeing the keratin fibers, and in particular human keratin fibers such as hair, which process comprises applying to the fibers one composition A containing, in a medium appropriate for dyeing at least one oxidation dye (base and/or coupler) or at least one direct dye, the colour being developed at alkaline, neutral or acid pH, with the aid of an oxidising composition B which is mixed just at the moment of use with the composition A or which is applied sequentially without intermediate rinsing, the nonionic compound defined in any of claims 1 to 5 being present in either the composition A or the composition B, or in both the compositions A and B.

26. A process according to claim 25, which process comprises applying the ready to use composition to the dry or wet hair, which ready to use composition is prepared at the moment of use from the compositions (A) and (B), leaving it to act for a resting time varying preferably from 1 to 60 minutes, and more preferably from 10 to 45 minutes, rinsing the fibers, then optionally shampooing and rinsing the fibers, and drying the fibers.

27. A process according to claim 25, which process comprises applying the ready to use composition to the dry or wet hair, which ready to use composition is prepared at the moment of use from the composition A' containing at least one oxidation dye and/or at least one direct dye, without the nonionic compound as defined in any of claims 1 to 5, composition A" containing at least one nonionic compound as defined in any one of claims 1 to 5 and an oxidising composition B, leaving it to act for a resting time varying preferably from 1 to 60 minutes, and more preferably from 10 to 45 minutes, rinsing the fibers, then optionally shampooing and rinsing the fibers, and drying the fibers.

28. A process according to any of claims 25 to 27, wherein the composition (A) and /or composition (B) further comprise at least one cationic or amphoteric polymer.

29. A device or kit having 2 compartments for dyeing keratin fibers, and in particular human keratin fibers such as hair, which comprises a compartment containing a composition Al comprising, in a medium appropriate for dyeing, at least one oxidation dye (base and/or coupler), or at least one direct dye, and another compartment containing a composition B 1 comprising, in a medium appropriate for dyeing, an oxidising agent, wherein one or more nonionic compounds as defined in any of claims 1 to 5 are present in either the composition Al or the composition B1, or in both of the compositions A1 and B1.

30. A device having 3 compartment for dyeing keratin fibers, and in particular human keratin fibers such as hair, which comprises a first compartment containing composition A2 comprising, in a medium appropriate for dyeing at least one oxidation dye (base and/or coupler), at least one direct dye, a second compartment containing composition B2 comprising, in a medium appropriate for dyeing, at least one oxidising agent, and a third compartment containing a composition C comprising, in a medium appropriate for dyeing, at least one nonionic compound as defined in any of claims 1 to 5, wherein the composition A2 and/or the composition B2 can also further comprise the nonionic compound as defined in any of claims 1 to 5.

31. Use of a dye composition according to any of claims 1 to 23 or a dyeing device or kit having a plurality of compartments according to claim 29 or 30, for dyeing keratin fibers such as human hair.
